# EUROPÄISCHE PATENTSCHRIFT

(11) **EP 1 121 481 B1**
(45) Veröffentlichungstag und Bekanntmachung des Hinweises auf die Patenterteilung: **27.04.2005**
(21) Anmeldenummer: 99946007.4
(22) Anmeldetag: 23.08.1999
(51) Int. Cl.: D04H 3/02, A61F 13/15, D04H 1/46

(54) **PERFORIERTER VLIESSTOFF UND VERFAHREN ZU DESSEN HERSTELLUNG**
PERFORATED BONDED FIBER FABRIC
NAPPE DE FIBRES PERFOREE ET PROCEDE DE FABRICATION DE LADITE NAPPE

(30) Priorität: 12.10.1998 DE 19846857
(43) Veröffentlichungstag der Anmeldung: 08.08.2001
(73) Patentinhaber: Carl Freudenberg KG, 69469 Weinheim (DE)
(72) Erfinder: GROITZSCH, Dieter, D-69493 Hirschberg (DE); SCHAUT, Gerhard, D-69502 Hemsbach (DE); KLEIN, Bernhard, D-69488 Birkenau (DE)
(86) Internationale Anmeldenummer: PCT/EP1999/006144
(87) Internationale Veröffentlichungsnummer: WO 2000/022218

(56) Entgegenhaltungen:
- EP-A- 0 215 684
- EP-A- 0 604 736
- EP-A- 0 815 819
- WO-A-98/23804
- US-A- 4 840 829
- US-A- 5 171 238
- US-A- 5 369 858

## Beschreibung

Körperflüssigkeiten absorbierende Hygieneprodukte, wie Kinderwindeln, Erwachsenenwindeln oder Damenbinden sind grundsätzlich aus einem absorbierenden Kern, einer abdichtenden Rückseite aus einer Folie oder einem Vlies/Folien-Laminat und einem körperseitigen, durchlässigen Flächengebilde aus einem dünnen, abriebbeständigen, weichen Vliesstoff oder einer vakuumperforierten Folie mit trichterförmigen, d.h. dreidimensionalen Öffnungen aufgebaut. Die vakuumperforierte Folie umschließt den absorbierenden Kern, wobei die größte Perforationsöffnung nach außen gerichtet, d.h. dem Körper zugewandt ist. Das Folienmaterial ist aus hydrophobem Thermoplast-Polymer, wie Polyäthylen, Polypropylen oder einem Copolymer aus Ethylen und Polyvinylacetat (EVA) aufgebaut. Dadurch wird erreicht, daß die Folienoberfläche von der Körperflüssigkeit einerseits nicht benetzt wird, die Körperflüssigkeit nur in Richtung Absorberkern geleitet wird und durch die sich nach innen verjüngenden Perforationen ein Rückschlag derselben z.B. bei Belastung, Bewegung, oder Druck verhindert wird. Bekanntermaßen enthält der Absorberkern gewöhnlich neben überwiegend Zellstoff auch Superabsorber-Partikel (SAP). Superabsorber-Polymere zeichnen sich dadurch aus, daß sie wäßrige Flüssigkeiten in großen Mengen aufnehmen können und dabei unter deutlicher Volumenzunahme einen Gelkörper mit mehr oder weniger geringen Gelfestigkeit bilden. Die Gegenwart von SAP hat den Vorteil, daß Gewicht eingespart und dadurch die Dicke des Absorberkerns verringert werden kann und daß die Flüssigkeit bei Druckbelastung nicht wieder abgegeben werden kann und dadurch Leckagen weitgehend verhindert werden können. SAP hat aber auch den Nachteil, daß es zum bekannten Gelblocking führt und zwar um so ausgeprägter, je höher sein Anteil ist. Unter Gelblocking versteht man den Effekt, daß Flüssigkeit nicht mehr weiter oder nur deutlich verlangsamt weitertransportiert werden kann. Durch geeignete Konstruktionen der absorbierenden Hygieneprodukte konnte auch dieses Problem gelöst werden. Es werden in diesem Fall Volumenvliesstoffe oder andere bei Flüssigkeitskontakt nicht blockierende, sehr offene Strukturen zwischen Absorberkem und Abdeckschicht positioniert. Diese Zwischenschicht nimmt Flüssigkeit sofort auf, d.h. entfernt sie spontan von der Windeloberfläche und verteilt sie gleichmäßig. Das Fluidmanagement wird durch solche Maßnahmen verbessert. Unter Fluidmanagement verstehen wir hier das Zusammenspiel vieler, oben teilweise schon genannter Einflußgrößen mit dem Ziel, ein möglichst hohes Wohlempfinden beim Tragen des Hygieneartikels am Körper zu erzeugen.

Als Flächengebilde für die körperseitige Umhüllung des absorbierenden Materials werden bekanntermaßen auch nichtperforierte Spinnvliesstoffe und Stapelfaservliesstoffe auf Basis von Polyolefinen eingesetzt.

Das Fluidmanagement für Urin bei Kinder- und Erwachsenen-Windeln und für Menstrualflüssigkeiten der Damenhygiene gilt als weit fortgeschritten bis ausgereift. Eine Windel der Zukunft sollte jedoch nicht nur fähig sein, Urin in optimaler Weise zu managen, sondern auch dünnflüssige Abgänge aus dem Darm. Nicht perforierte Abdeckvliesstoffe erwiesen sich für diesen Zweck als ungeeignet. Die betreffende Körperflüssigkeit ist ein mehrphasiges System mit Festpartikeln in unterschiedlicher Form und Konsistenz mit der Tendenz zur Phasentrennung, insbesondere an aktiven Oberflächen oder Oberflächen mit Filtrations- und Abscheidewirkung. Für diese Flüssigkeiten wird nachstehend der Ausdruck Darmflüssigkeiten verwendet. Es hat sich gezeigt, däß nicht perforierte Vliesstoffe ungeeignet sind, Darmflüssigkeiten vollständig durchzulassen und an den Absorberkem weiterzugeben. Vielmehr besteht die Tendenz, daß feste und / oder hochviskose Anteile der Darmflüssigkeit sich auf der Windeloberfläche durch Separation ablagern und ggfs. wie eine Sperrschicht für nachkommende Körperflüssigkeit mit dünnflüssigerer Konsistenz wirken. Sowohl die Separation der gröberen Bestandteile an sich als auch die damit verbundene Blockade für weiteren Fuidtransport sind gravierende Nachteile konventioneller Windeln. Es sind daher zahlreiche Lösungsvorschläge zum besseren Darmflüssigkeitsmanagement gemacht worden, die alle darauf beruhen, daß perforierte Topsheets (Abdeckvliesstoffe) eingesetzt werden müssen. Die Perforationen sollten dabei klar ausgebildet sein. Querverstrebungen einzelner Fasern oder Faserscharen bzw. irgendwelche Faserbrücken erwiesen sich als nicht günstig. Über die perforierten Topsheets hinaus sollten die Windelkonstruktion und die Gestaltung des zwischen dem Abdeckvliesstoff und dem Absorber-Kern gelegenen offenstrukturierten Vliesstoffes der besonderen Konsistenz und der damit verbundenen Eigenheiten der Darmflüssigkeit angepaßt werden.

Sowohl zahlreiche Perforationsmethoden als auch Vliesstoffe und Vliesstoffkomposite sind bekannt. In EP-A-0 215 684 wird die Erzeugung von Perforationen in Vliesstoffen mit Hilfe der Wasserstrahltechnik beschrieben. Als Ablagemedium für die Fasern und Wasserstrahlbehandlung werden nicht die bekannten Siebe verwendet, sondern dieselben ersetzt durch Entwässerungszylinder, in die Erhebungen eingelassen sind. Diese sind für eine klare Perforationen verantwortlich. In der US 5,628,097 werden eine andere Perforationsmethode und perforierte Produkte beschrieben, bei der der Vliesstoff mit Hilfe von Ultraschall oder thermisch in Längsrichtung geschlitzt und durch Passagen eines aus zwei ineinandergreifenden Riffelwalzen bestehenden Walzenpaares in Querrichtung gestreckt wird. Die Schmelzstellen-Schlitze werden dadurch getrennt und zu Perforationen geöffnet. Beschrieben sind Vliesstoffe aus Stapelfasern und Endlosfilamenten, Meltblown-Vliesstoffe und Verbundstoffe aus Stapelfasern und Endlosfilamenten mit Meltblown, die beispielsweise als SM (für Komposit Spunbond/Melt-blown) oder SMS (für Komposit Spunbond/Meltblown/**Spunbond**) bezeichnet werden.

Von einem perforierten Vliesstoff im Hygienebereich wird nicht nur Darmflüssigkeitsmanagement sondern auch ein möglichst hoher Weißgrad bzw. eine hohe Deckkraft und eine sehr hohe Weichheit zumindest auf der körperzugewandten Seite verlangt. Es ist bekannt, daß beide Eigenschaften von der Geschmeidigkeit und Weichheit der eingesetzten Fasern selbst abhängen.

Diese sind um so höher, je niedriger der Fasertiter ist, so daß es sich anbietet, Fein-, Feinst- oder gar Ultrafeinstfasern zu verwenden. Ultrafeine Fasern werden auch als Mikrofasem bezeichnet. Diese können auf Gewebe oder Vliesstoffen basieren. Auch Meltblown-Vliesstoffe bestehen aus Mikrofasem im Größenbereich von ca. 1 -10 microns.

Es ist eine Kinderwindel vom Hersteller Unicharm bekannt, die mit einem perforierten Vliesstoff abgedeckt ist, der nach der bereits oben kurz beschriebenen speziellen Wasserstrahlperforationsmethode hergestellt wurde und aus einem Komposit PP/PE-Spunbond und einer PP-Meltblown-Schicht besteht. Mit dieser Verbundstoff-Konstruktion werden zwar ein Beitrag zum besseren Management der Darmflüssigkeit, eine gute Weichheit an der Meltblownseite (= Körperseite) und eine hohe Deckkraft geleistet. Diese Verbundkonstruktion und deren Herstellungsmethode weisen jedoch auch gravierende Nachteile auf. Die Meltblown-Schicht leistet keinen oder nur einen völlig unbedeutenden Beitrag zur Gesamtfestigkeit bzw. Gesamtintegrität des Verbundes. Die Gewichte liegen deutlich über den heute üblichen. Eine Gewichtsreduktion auf unter ca. 30 g/m² erscheint aufgrund der hohen Festigkeitsanforderungen in der Maschinenrichtung zur Windelfertigung als nicht möglich. Der hohe Materialeinsatz ist kostenintensiv. Die Meltblown-Schicht für sich allein betrachtet ist nicht abriebbeständig und muß zusätzlich zur Wasserstrahlbehandlung noch thermisch mit dem Spunbond-Trägervliesstoff verankert werden, um Delaminierungstendenzen zu verhindern. Dies wiederum verlangt Bikomponenten-Fasem (conjugated fibers) mit einer zentrischen oder exzentrischen Mantelkomponente aus niedriger schmelzendem Polymer als dem der Meltblown-Schicht. Dennoch erreicht dieser perforierte SM-Verbund an der weichen M-Seite bei weitem nicht die Abriebbeständigkeit eines PP-Spunbonds oder PP-prägegebundenen Stapelfaservliesstoffes, wie sie heute in Windeln und Damenbinden eingesetzt werden. Bei anderen Anwendungen, wie abdichtenden Windelhosenmanschetten oder OP-Vliesstoffen, bei denen Abriebbeständigkeit bzw. Lintfreiheit gefordert wird, kann nur SMS eingesetzt werden. Mit einer solchen Abdeckung der Meltblown-Schicht zur Körperseite würden die Vorteile der Meltblown-Schicht nicht mehr zum Tragen kommen.

**Aus dem Dokument US 4,840,829 sind Vliesstoffe mit einem Flächengewicht von 10 bis 150 g/m**^{**2**} **bekannt, die aus Stapelfasern mit einer Länge von 20 bis 100 mm und einem Titer von 0,555 bis 16,65 dtex hergestellt werden. Diese Vliesstoffe besitzen kreisförmigen oder elliptischen Öffnungen, die durch Wasserstrahlbehandlung auf einer mit Erhebungen versehenen Unterlage erhalten werden.**

**Weiterhin sind aus dem Dokument WO98/23804 verfestigte Vliesstoffe und Verfahren zu ihrer Herstellung bekannt, die aus Mehrkomponentenfasern bestehen und die bei ihrer Verfestigung zu einem Vliesstoff in ihre einzelnen Komponentenfasern getrennt und verwirbelt werden**

Es ist Aufgabe der Erfindung, einen perforierten Vliesstoff zu schaffen, der beim Darmflüssigkeitsmanagement bisherigen Vliesstoffen überlegen ist, den Anforderungen an hohe Opazität und höhere Weichheit und Zartheit an der körperseitigen Oberfläche nachkommt, einen zwei- oder mehrschichtigen Aufbau unnötig macht und mit einem Fasermaterial-Gewicht auskommt, das deutlich unter dem von derzeit in Windeln und Damenbinden eingesetzten perforierten Vliesstoffen liegt. Außerdem ist es Aufgabe der Erfindung, das Darmflüssigkeitsmanagement ohne Beeinträchtigung des Urin-Managements zu verbessern. Weiterhin ist es Aufgabe der Erfindung, den Fluid-Durchgang durch den perforierten Vliesstoff ohne den Einsatz von Detergentien zu erreichen bzw. deren Einsatzmenge auf einen Bruchtteil der in nichtperforierten Hüllvliesstoffen üblichen Mengen herabzusetzen.

Die Aufgaben werden erfindungsgemäß gelöst durch einen perforierten Vliesstoff mit einem Flächengewicht von 7 bis 25 g/m2 aus miteinander verschlungenen endlosen Mikrofaserfilamenten, mit einem Titer im Bereich von 0,05 bis 0,40 dtex, die aus mindestens zwei thermoplastischen Polymeren mit unterschiedlicher Hydrophobizität auf**gebaut sind** und einem Filamentquerschnitt in **Pie- oder Hollow-Pie-Form aufweisen,** aus **denen** die **Splitt-**Filamente freigesetzt worden sind, wobei die Perforationen klar ausgebildet und frei von **Splitt-**Faserfilamenten sind.

Die erfindungsgemäßen Vliesstoffe zeigen trotz extrem niedrigem Gewicht sehr hohe Festigkeiten und aufgrund der niedrigen Fasermasse sehr klare Lochstrukturen. Dadurch ist es möglich, den schnellen Durchgang von Körperflüssigkeiten, insbesondere Darmflüssigkeiten, ohne oder nur mit geringem Zusatz von oberflächenaktiven Stoffen mit niedriger Oberflächenspannung (Netzmittel) zu gewährleisten und für Windeln und Damenbinden eine trockene Topsheet-Oberfläche zu erzeugen.

Die unterschiedlichen Filamente weisen jeweils einen Titer im vorstehend angegebenen Bereich auf. Die Perforationen sind vorzugsweise regelmäßig angeordnet und weisen eine Einzellochfläche von 0,01 bis 0,60 cm² auf.

Der erfindungsgemäße perforierte Vliesstoff weist vorzugsweise einen Strike Through-Wert nach einer Minute von weniger als 3 sec. auf. Die Höchstzugkraft in Längsrichtung beträgt vorzugsweise mindestens 30 N/5 cm. Der Rewet-Wert beträgt vorzugsweise weniger als 0,5 g.

Zum Aufbau des Vliesstoffes können beispielsweise zwei unterschiedliche Filamente aus thermoplastischem Polymeren in einem Gewichtsverhältnis im Bereich von 20:80 bis 80:20 eingesetzt werden. Nachstehend wird der Aufbau des Faservlieses anhand zweier Filamente F1 und F2 erläutert.

Die Erfindung betrifft auch ein Verfahren zur Herstellung derartiger perforierter Vliesstoffe durch Ablegen von splittbaren pie- oder hollow pie-Endlosfasern, deren Querschnitt mindestens zwei unterschiedliche thermoplastische Polymere mit unterschiedlicher Hydrophobizität in einer abwechselnden Kuchenstückanordnung aufweist, zu einem Vliesstoff, nachfolgendes Splitten und Verschlingen der Faser zu verschlungenen Endlosfilamenten mit Hilfe von Hochdruck-Wasserstrahlen, und nachfolgendes Perforieren des gebildeten Vliesstoffes mit Hochdruck-Wasserstrahlen.

Dabei erfolgt das Perforieren vorzugsweise auf Entwässerungs- und Lochbildungstrommeln, die Erhebungen auf der Oberfläche aufweisen.

Nachstehend werden zunächst die zur Herstellung des erfindungsgemäßen Vliesstoffes eingesetzten Polymere und danach das Herstellungsverfahren näher erläutert.

Von den beiden Faserpolymeren F1 und F2 ist mindestens eines der beiden hydrophob und stammt vorzugsweise aus der Reihe der Polyolefine, wie Polyethylen, Polypropylen oder Copolymere davon, bei denen eines der beiden im Überschuß vorhanden ist. Das andere kann sowohl hydrophob als auch hydrophil sein, ist jedoch vorzugsweise nicht hydrophil, sondern weniger hydrophob als Polypropylen. Das stärker hydrophobe Faserpolymer sei hier mit F1 und das schwächer hydrophobe Faserpolymer mit F2 bezeichnet. F1 wird vorzugsweise aus Polypropylen (PP) oder Polyethylen (PE) oder verschnitten der beiden bestehen. F2 kann beispielsweise eine Faser aus der Reihe der Polyester, wie Polyethylenterephthalat, Potybutylenterephthalat, Polypropylenterephthalat oder ein Copolyester davon und PE sein. Sowohl F1 als auch F2 unterliegt, was die Polymerauswahl angeht ansonsten keiner Beschränkung, außer der, daß sie mit den bekannten Spinnvliesverfahren zu konjugierten Fasern versponnen werden können.

Von F1 und F2 können beide oder eines der beiden aus thermoplatischen Elastomeren bestehen. Beispiele für elastische Polyolefine für Spinnvliesstoffe finden sich in EP-A-O 625 221 und für metallocen-katalysiertes LLDPE in EP-A-O 713 546, in der auch Vertreter für die schwächer hydrophoben Elastomere, wie Polyurethane, Ethylen-Butylen-Copolymere, Poly (Ethylen-Butylen)-Styrol-Copolymere (Kraton), Poly-Adipat-Ester und Polyetherester-Elastomer (Hytrel) beschrieben sind. Von diesen Elastomeren ist bekannt, daß Spinnvliesstoffe im Meltblown- oder SMS-Kombinationen ersponnen werden können. Der Einsatz solcher Elastomere in F1 und/oder F2 erhöht die Weichheit und Geschmeidigkeit des perforierten Mikrofaservliesstoffes. Es hat sich außerdem gezeigt, daß nur perforierte Vliesstoffe, die aus miteinander verschlungenen Mikrofaserendlosfilamenten bestehen, die hervorragenden Eigenschaften hinsichtlich Fluid-Management zeigen. Perforierte Vliesstoffe aus in gleicher Weise miteinander verschlungenen Mikrofaser-Stapelfasern erreichen diese verbesserten Eigenschaften nicht. Allein der Verarbeitung auf Windelmaschinen (hohe Zugkraftbeanspruchung in Maschinenrichtung). wegen, müßte schon das Gewicht desselben gegenüber dem Endlosfaservliesstoff durchschnittlich verdreifacht werden, mit deutlichen Einbußen in Perforationsgüte, Geschmeidigkeit, Weichheit, Abriebbeständigkeit und Fluidmanagement.

Auch Zusätze von Ingredienzen zur Faserpolymerschmelze in Form von Masterbatches zum Zwecke der Antistatik-Ausrüstung, der Spinnfärbung, der Mattierung, der Weichmachung, Klebrigmachung und Flexibilisierung der Faser, Erhöhung und Erniedrigung der abweisenden Eigenschaften gegen Flüssigkeiten (wie Wasser, Alkohole, Kohlenwasserstoffe, Öle), Fette und multi-disperse Systeme, wie Darmflüssigkeiten und andere flüssige Körperausscheidungen, wie Urin und Menstrualflüssigkeit sind möglich.

Ingredienzien, welche die Grenzflächenspannung an der Mikrofaseroberfläche verändern, können auch durch nachträgliche Applikation nach der Generierung bzw. Freisetzung der Mikrofaserfilamente in dem bereits perforierten Vliesstoff aufgetragen werden. Solche Stoffe sind beispielsweise Netzmittel in Wasser gelöster oder dispergierter Form, mit denen heute viele Windelabdeck-Spinnvliesstoffe zum Zwecke des besseren Urin-Managements ausgerüstet **sind**.

Die Vliesstoffe gemäß der vorliegenden Erfindung kommen jedoch vorzugsweise ohne solche Netzmittel bzw. mit nur einem Bruchteil der bisher üblichen Applikationsmenge aus. Die Ausgestaltung der Perforationen, d.h. deren Lochgröße, deren Form, der Anordnung der einzelnen Perforationen zueinander (z.B. auf Lücke oder in Reihe) und der offenen Fläche einerseits sowie die extrem hohe Geschmeidigkeit der aus verschlungenen Endlosmikrofaserfilementen bestehenden Stege (Bereich zwischen den Perforationen) und deren sehr niedriges Gewicht erlauben diese Netzmittelreduktion bis zu völliger Einsparung.

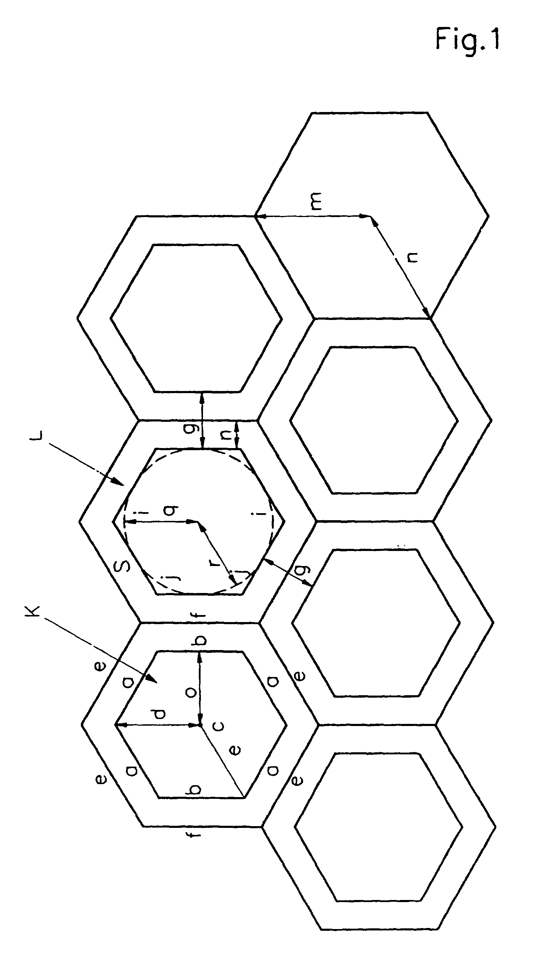

Die Zeichnung mit den Fig. 1 bis 6 erläutert die Erfindung zusätzlich.

In den Fig. 1 bis 6 wird die Form der einzelnen Öffnungen K und deren Anordnung in einem Flächengebilde angezeigt. In Fig.1 ist K eine idealisiert dargestellte Öffnung in Form eines gleichseitigen Sechseckes, wobei die Seitenlänge a mit b identisch ist. Der Abstand o ist der kürzeste Abstand zwischen dem Zentrum c der Öffnung K und der Kante a. Die Kanten a und b stehen jeweils im konstanten Abstand 9 zu jedem benachbartem K. Um die einzelnen Öffnungen K läßt sich jeweils, parallel zu a und b ausgerichtet ein größeres gleichseitiges Sechseck mit den Kanten e und f legen. In Fig. 1 ist e = f. Dadurch entsteht eine bienenwabenförmige Anordnung der Öffnungen K. Die Kanten a und b einer Öffnung K sind jeweils parallel zu den benachbarten Kanten a und b der benachbarten Öffnungen K ausgerichtet. Der Abstand h = 0,5 9. Die Spitzen an den Berührungskanten a mit a bzw. a mit b sind in dem Vliesstoff in abgerundeter Form vorhanden. Diese Abrundungen i und j der Spitzen sind in Fig.1 für den Fall i = j wiedergegeben. Durch diese Abrundungen verkürzen sich die ursprünglichen Abstände d zu e des Hexagons zu q und r. Im Falle der Fig. 1 ist wieder q = r.

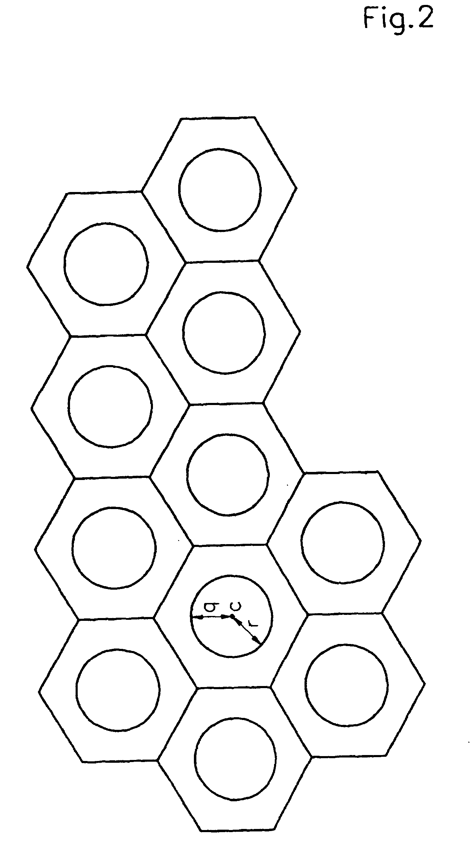

Alle Abrundungen i und j können im Extremfall so stark ausgeweitet sein, daß sich für K eine kreisrunde Form ergibt, wie in Fig. 2 dargestellt ist.

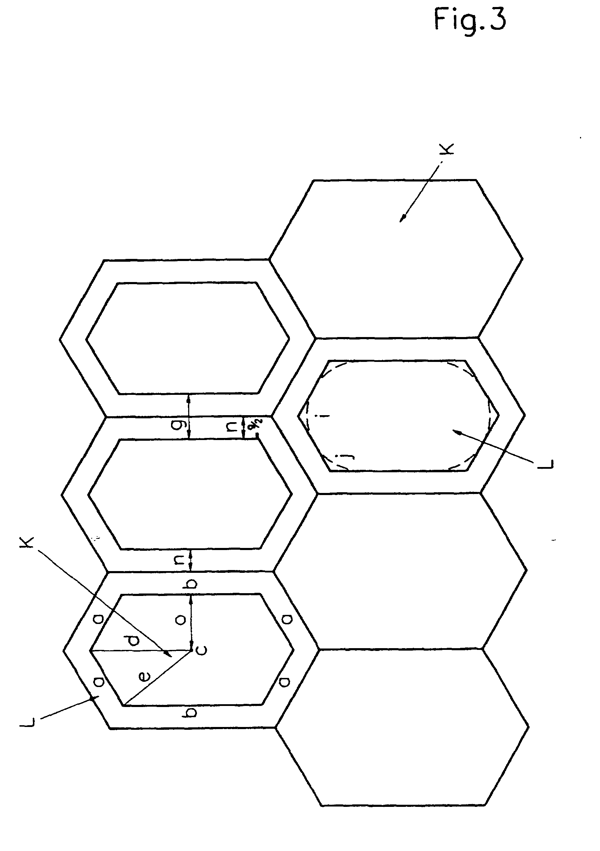

Die Öffnungen K der Fig. 3 unterscheiden sich von denjenigen der Fig. 1 nur dadurch, daß b deutlich länger als a ist und die Abrundung i stärker ausgeprägt ist als j.

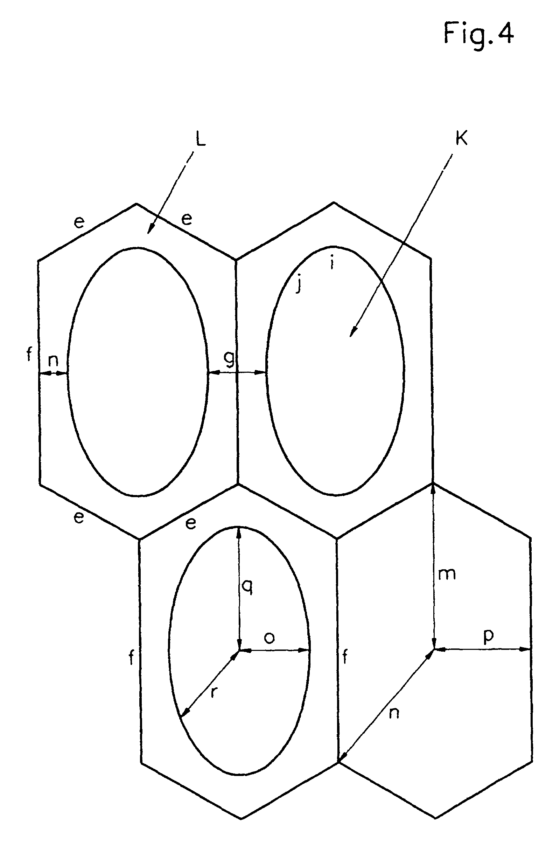

Die Abrundungen i und j können im Extremfall so weit ausgedehnt sein, daß aus dem sechseckigen K eine elliptische Form resultiert, wie in Fig.4 dargestellt.

Hexagonale Formen der Öffnungen K oder solche, die sich durch Abrundungen daraus ergeben und die Anordnung derselben, wie sie in den Fig. 1 bis Fig. 4 dargestellt sind, haben sich für das Fluid-Management als besonders bevorzugenswert erwiesen. Insbesondere bei gleichseitig hexagonalen Öffnungen K und deren abgerundeten Ableitungen hat die Körperflüssigkeit immer den kürzesten Weg von der Windeloberfläche in das Windelinnere.

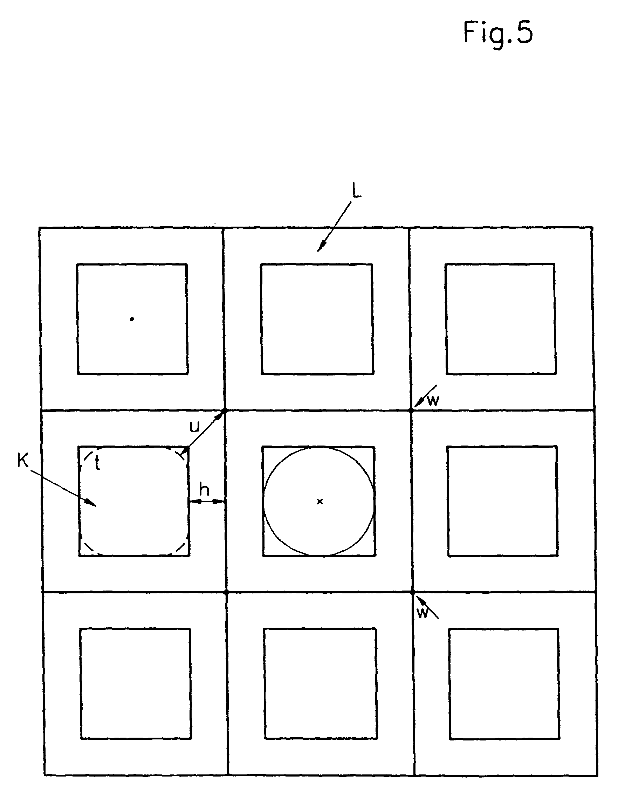

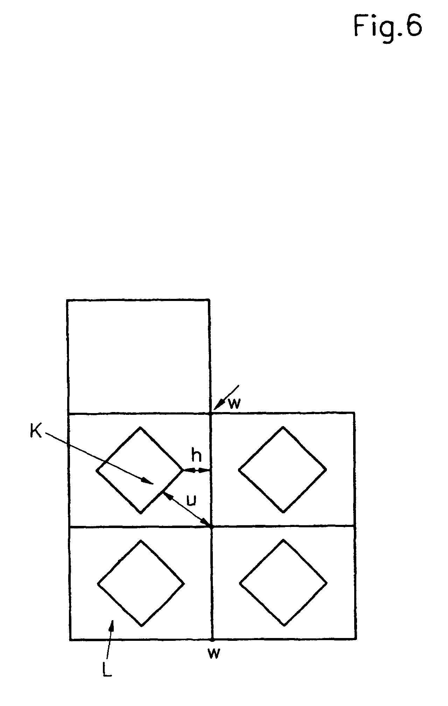

Die Erfindung beschränkt sich jedoch nicht auf solche regelmäßigen Formen und Anordnungen. Auch andere Vielecke für K und deren abgerundete Abkömmlinge sind denkbar sowie auch unregelmäßige Verteilungen solcher oder anderer Öffnungen. Weniger geeignet sind allerdings solche Öffnungen und Anordnungen derselben, die dem Teil der ausgeschiedenen Körperflüssigkeit, der am weitesten von den Öffnungsrand entfernt ist, einen Hinderungsgrund geben, schnell durch die Öffnungen K abfließen zu können. Solche Anordnungen werden beispielsweise in Fig. 5 und 6 wiedergegeben.

Die Entfernung von dem weitest entfernten Punkt w bis zur (abgerundeten) Ecke des Viereckes ist deutlich größer als die Entfernung h. Das Verhältnis u/h von maximaler Entfernung zur Öffnung K zu minimaler Entferung sollte im Idealfall 1/1 und im schlechtesten Fall nicht über 2/1 liegen.

Die Einzellochfläche bewegt sich im Bereich von 0,01 bis 0,60 cm², vorzugsweise zwischen 0,04 bis 0,40 cm². Die einzelnen Löcheröffnungen können alle die gleiche Form und einheitlich die gleiche Lochfläche besitzen. Sie können aber beide oder nur eine der beiden unterschiedlich sein, jedoch die o.g. Lehre von u/h kleiner/gleich 2/1 beachtend.

Die offene Lochfläche liegt im Bereich von 8 bis 40%, vorzugsweise von zwischen 12 bis 35 %.

Die mikrofeinen, verschlungenen Endlosfilamente S bilden den Rahmen L für die Öffnungen. Der perforierte Vliesstoff kann, wie vorstehend erwähnt, oberflächenaktive Wirksubstanzen, die ihm eine auswachbare, verzögert auswaschbare oder bleibende Hydrophilie verleihen, enthalten. Diese werden zweckmäßigerweise nach der Wasserstrahlperforation im Naß-in-Naß-Verfahren aufgetragen. Die Auftragsmenge liegt zwischen 0 bis 0,60 Gew -%, bezogen auf das Vliesstoffgewicht, vorzugsweise zwischen 0 und 0,20 %. Die Dosierung richtet sich nach der Fläche der einzelnen Löcher und der offenen Gesamtfläche. Je größer beide sind, um so stärker kann der Gehalt an derartigen Surfactants herabgesetzt werden. Aus Gründen optimaler Bioverträglichkeit wird ein Gehalt an Surfactants von 0 % angestrebt.

Als besonders vorteilhaft hat es sich erwiesen, das oberflächenaktive Agenz (surfactant) nicht gleichmäßig über den gesamten Rahmen zu verteilen, sondern auf die unmittelbare Nachbarschaft zur Lochperipherie zu beschränken. Von dieser Stelle geht dann zwangsweise eine zur Perforationen hin gerichtete Saugwirkung auf das Fluid aus. Das multidisperse Fuidsystem erleidet dann keine Entwässerung bzw. Phasentrennung. Ein Verstopfen der Perforationen und Ablagerungen auf dem Rahmen werden verhindert. Die zwischen Absorbent Core und Topsheet eingelagerte Fuidaufnahme- und Verteilerschicht, die ebenfalls benetzend eingestellt ist, fördert zusätzlich die sofortige Entfernung der Körperflüssigkeit von der Windeloberfläche.

### Herstellung des perforierten Vliesstoffes (Topsheet)

Das Verfahren besteht darin, daß eine splittbare pie- oder hollow pie-Faser mit Hilfe der Spinnvliestechnologie zu einem Vliesstoff aus Endlosfilamenten abgelegt wird. Die Querschnitte der ungesplittet aus der Düse austretenden Fasern bestehen aus den zwei unterschiedlichen Polymerkomponenten F1 und F2, die sich in abwechselnder Reihenfolge wie Kuchenstücke aneinanderreihen (Normalfall aus 4 bis 16 solcher Kuchenstücke). Als Voraussetzung für eine sich anschließende Splittung sollten vorzugsweise solche meist 2 polymer-chemisch stark unterschiedliche Komponenten eingesetzt werden, die an ihren gemeinsamen Grenzflächen nur eine möglichst geringe Haftung aufweisen. Es können aber auch chemisch ähnliche Polymerkomponenten verwendet werden, wie beispielsweise Polyethylenterephthalat und ein Copolyester oder Polypropylen und Polyethylen, sofern Maßnahmen getroffen worden sind, die Haftung an den Grenzflächen der beiden zum Beispiel durch Zusatz an Trennmittel zumindest in einer Faserpolymerkomponente herabzusetzen. Ist die Splittfaser innen mit einem (runden) Hohlraum versehen, spricht man von einer sog. Hollow-Pie-Faser, ansonsten von einer Pie-Faser.

Der Titer der Endlosfilamente in dem Spinnvliesstoff beträgt vor dem Splitten in der Regel 1,0 bis 4,0 dtex, vorzugsweise 1,6 bis 3,3 dtex . Anschließend werden die Endlosfilamente des Spinnvliesstoffes mit bekannten Methoden der Hochdruck-Wasserstrahltechnik (siehe z.B. EP-A-O 215 684) in einer ersten Nachbehandlungsstufe miteinander verschlungen und gleichzeitig in die Kuchenbestandteile aufgesplittet. Bei einer Pie-Faser mit einem Titer von 1,6 dtex und insgesamt 16 Segmenten, die sich aus je 8 Segmenten der beiden Faserpolymere zusammensetzen, liegen also nach der Splittung Mikrofasem in einem Titer von 0,10 dtex vor. Nachdem es sich bei der Erfindung um einen sehr leichten Vliesstoff handelt, ist es vorteilhaft, als Support, auf den der Vliesstoff aufgelegt wird, kein Sieb oder kein Support mit Perforationen zu verwenden, sondern einen völlig unperforierten Support. Dadurch kann durch Reflexion der Wasserstrahlen an diesem Support deren Prallwirkung ausgenutzt und damit der Energieverlust minimiert werden.

Nach der Perforation wird entweder getrocknet oder zuvor zweckmäßigerweise in einer Naß-in Naß-Applikationsmethode vor der Trocknung Tensid zum Zwecke der oberflächigen Hydrophilierung aufgetragen. Dies kann nach bekannten Methoden der Vollbadimprägnierung, des einseitigen Pflatschens, des Aufstreichens oder des Druckens geschehen. In einer besonderen Ausgestaltungform wird das Tensid (Netzmittel) musterförmig aufgedruckt, in der Weise, daß nur die Grenzbereiche des Faserrahmens zur Perforation betroffen sind. Dies bedarf der Erstellung spezieller Druckschablonen, die dem Perforationsmuster angepaßt sein müssen, und besonderer Kontrollmaßnahmen zur Erhaltung der Konturenschärfe des Netzmitteldruckes während der Fertigung.

### Beispiel: 1

Es wird ein Spinnvliesstoff mit einem Flächengewicht von 13 g/m2, der zu 100% aus einer Pie-Faser mit einem Fasertiter von 1,6 dtex besteht, auf einern Sieb abgelegt. Die Pie-Faser besteht in ihrem Querschnitt aus abwechselnd je 8 Polypropylen-Segementen und je 8 Polyethylenterephthalat-Segmenten. Die Größe der einzelnen Polypropylen-Segmente ist so gewählt, daß der Gewichtsanteil des Polypropylens 30 % und des Polyethylenterephthalats 70% ausmacht.

Der ungesplittete Endlosfilament-Vliesstoff wird auf ein 100 mesh Entwässerungssieb gelegt und mit einem Wasserstrahldruck von 180 mbar verfestigt, und die Endlosfilamente werden jeweils in ihre 8 Mikrofasersegmente aus Polypropylen und 8 Mikrofasersegmente aus Polyethylenterephthalat aufgesplittet.

Nach der Splittung entstehen jeweils die gleiche Anzahl an Mikrofasersegmenten aus Polypropylen und Polyethylenterephthalat. Die Mikrofasersegmente aus Polypropylen weisen einen Einzeltiter von 0,06 dtex und die Segmente aus Polyethylenterephthalat einen Einzeltiter von 0,14 dtex auf. Die Umrechnung von dtex in Faserdurchmesser (idealisiert auf runden Querschnitt) ergibt für Polypropylen (Dichte von 0,91 g/cm³) eine Wert von 2,36 micron und für Polyethylenterephthalat (Dichte von 1,37 g/cm³) einen Wert von 4,42 micron.

Nach dem Aufsplitten der Faser durch Wasserstrahlen wird das Flächengebilde einer Perforation ebenfalls mit Hilfe von Hochdruckwasserstrahlen mit einem Druck von 70 kg/cm² unterzogen. Hierzu werden die in EP-A-0 215 684 beschriebenen Entwässerungs- und Lochbildungstrommeln mit Erhebungen auf der Oberfläche der Trommel anstelle der sonst üblichen Entwässerungssiebe eingesetzt.

Nach der Trocknung entsteht ein sehr weicher, anschmiegsamer Vliesstoff mit klar ausgebildeten Perforationen. Die einzelnen Löcher der Perforation sind alle (idealisiert) kreisförmig ausgebildet und von gleicher Größe. Die Anordnung der Löcher erfolgt in einem orthogonalen Gitter mit einem Gitterabstand a, wobei jeweils flächenzentriert ein weiteres Gitter mit Löchern überlagert ist.

Der Radius r beträgt durchschnittlich 1,4 mm und der Abstand a = 6,0 mm. Die offene Fläche OF beträgt 34 %, bezogen auf die Gesamtfläche.

Von den perforierten Vliesstoff wurden die Höchstzugkraft in Längsrichtung nach EDANA 20.289, die Liquid Strike Through Time nach EDANA 150.3-96 und der Coverstock Wet Back (auch Rewet genannt) nach EDANA 151 .1-96 gemessen.

Der Strike Through wurde nach einer Wartezeit von je 1 Minute insgesamt 2 mal wiederholt, ohne die Filterpapier-Lagen zu wechseln. Die angegebenen Werte sind jeweils die Mittelwerte aus insgesamt 3 Einzelmessungen.

### Ergebnisse:

### Höchstzugkraft in Längsrichtung: 32,3 N/5 cm

| | | |
|---|---|---|
| 1. Strike Through (sec) | 2. Strike Through (sec) | 3. Strike Through (sec) |
| unmittelbar | nach 1 Minute | nach 1 weiteren Minute |
| 1 ,82 | 2,42 | 2,44 |

### Rewet: 0,09 g

### Beispiel: 2

Der perforierte Vliesstoff aus Beispiel 1 wurde im Foulard mit der sog. Vollbadmethode mit einer wäßrigen Emulsion eines nichtionischen Netzmittel auf der Basis von Polysiloxan getränkt. Die Auftragsmenge fest betrug nach der Trocknung 0,042 Gew -%. Mit diesem Muster wurden folgende Prüfergebnisse erzielt:

### Höchstzugkraft in Längsrichtung: 30,2 N/5 cm

| | | |
|---|---|---|
| 1. Strike Through (sec) | 2. Strike Through (sec) | 3. Strike Through (sec) |
| unmittelbar | nach 1 Minute | nach 1 weiteren Minute |
| 1,58 | 2,10 | 2,11 |

### Rewet: 0,31 g

### Vergleichsbeispiel: 1

Auf einen prägegebundenen Spinnvliesstoff aus Polypropyplen mit Endlosfilamenten des Titers 2,2 dtex und einem Flächengewicht von 10 g/m² wurde eine Meltblown-Lage aus 20 g/m² aufgesponnen. Der durchschnittliche Durchmesser der die Meltblown-Schicht aufbauenden Mikrofasem betrug 3,82 micron. Die Verschweißfläche des prägegebundenen Spinnvliesstoffes betrug 5,2 %.

Dieses zweilagige Laminat wurde entsprechend der in Beispiel 1 beschriebenen Methode wasserstrahlvemadelt und anschließend auf einem konventionellen 20 mesh Siebband perforiert. Die offene Fläche errechnete sich auf 18,4 % . Dieser zweilagige Vliesstoff war ebenfalls sehr weich, erbrachte jedoch deutliche Defizite hinsichtlich Höchstzugkraft und Strike Through im Vergleich zu den in Beispiel 1 und 2 gemessenen Prüfwerten. Strike Through und Rewet wurden jeweils auf der PP-Meltblown-Seite gemessen.

### Höchstzugkraft in Längsrichtung: 25,4 N/5 cm

| | | |
|---|---|---|
| 1. Strike Through (sec) | 2. Strike Through (sec) | 3. Strike Through (sec) |
| unmittelbar | nach 1 Minute | nach 1 weiteren Minute |
| 3,81 | 4,92 | 4,96 |

### Rewet 0,10 g

Die Strike Through-Werte sind für ein Topsheet deutlich zu hoch.

### Vergleichsbeispiel: 2

Auf das Muster aus Vergleichsbeispiel 1 wurde 0,40% nichtionisches Netzmittel auf Basis von Polysiloxan aufgetragen. Wie die Meßergebnisse zeigen, kann dadurch zwar der Strike Through zwar deutlich gesenkt werden, der Rewet erhöht sich aber unverhältnismäßig stark. Eine so hohe Rücknässung kann in einer Windel nicht akzeptiert werden.

### Ergebnisse:

### Höchstzugkraft in Längsrichtung: 24,6 N/5 cm

| | | |
|---|---|---|
| 1. Strike Through (sec) | 2 Sinke Through (sec) | 3. Strike Through (sec) |
| unmittelbar | nach 1 Minute | nach 1 weiteren Minute |
| 1 ,23 | 2,35 | 2,40 |

### Rewet: 2,35 g

Die Meltblown-Schicht verleiht dem Topsheet eine hohe Weichheit. In Gegenwart von Netzmittel wirkt diese Meltblown-Schicht jedoch wie ein Schwamm. Eine solche Konstruktion erwies sich somit zur Abdeckung einer Sauglage als ungeeignet.

### Vergleichsbeispiel: 3

Der in Vergleichsbeispiel 1 beschriebene 2-lagige Aufbau wird einer Wasserstrahlbehandlung entsprechend Beispiel 1 unterzogen.

Der durchschnittliche Radius r der Löcher nach der Wasserstrahlperforation betrug r = 1,28 mm. Der Abstand a blieb unverändert bei a = 6,0 mm.

Es ergibt sich eine offene Fläche 0F = 28,6 %.

### Ergebnisse:

### Höchstzugkraft in Längsrichtung: 24,2 N/5 cm

| | | |
|---|---|---|
| 1 Strike Through (sec) | 2. Strike Through (sec) | 3 Strike Through (sec) |
| unmittelbar | nach i Minute | nach 1 weiteren Minute |
| 2,93 | 3,78 | 3,84 |

### Rewet: 0,10 g

Die Strike Through-Werte sind wiederum zu hoch.

## Patentansprüche

1. Perforierter Vliesstoff mit einem Flächengewicht von 8 bis 17 g/m² aus miteinander verschlungenen endlosen Mikrofaserfilamenten mit einem Titer im Bereich von 0,05 bis 0,40 dtex, die aus mindestens zwei thermoplastischen Polymeren mit unterschiedlicher Hydrophobizität **aufgebaut sind** und einen Filamentquerschnitt in **Pie- oder Hollow-Pie-Form aufweisen,** aus **denen** die **Splitt-**Filamente freigesetzt worden sind, wobei die Perforationen klar ausgebildet und frei von **Splitt-**Faserfilamenten sind.

2. Perforierter Vliesstoff nach Anspruch 1, **dadurch gekennzeichnet, daß** die Perforationen regelmäßig angeordnet sind und eine Einzellochfläche von 0,01 bis 0,60 cm² aufweisen.

3. Perforierter Vliesstoff nach Anspruch 1 oder 2, **dadurch gekennzeichnet, daß** im Vliesstoff das Verhältnis von maximaler Entfernung von Punkten auf der Vliesstoffoberfläche zur nächsten Perforation zur minimalen Entfernung 1:1 bis 2:1 beträgt.

4. Perforierter Vliesstoff nach einem der Ansprüche 1 bis 3, **dadurch gekennzeichnet, daß** die offene Lochflache 8 bis 40 % beträgt.

5. Perforierter Vliesstoff nach einem der Ansprüche 1 bis 4, **dadurch gekennzeichnet, daß** der perforierte Vliesstoff aus Polyolefin- und Polyesterfilamenten in einem Gewichtsverhältnis im Bereich von 20:80 bis 80:20 aufgebaut ist.

6. Perforierter Vliesstoff nach einem der Ansprüche 1 bis 5, **dadurch gekennzeichnet, daß** der Vliesstoff mit 0 bis 0,60 Gew %, bezogen auf das Vliesstoffgewicht, mindestens einer oberflächenaktiven Wirksubstanz imprägniert ist

7. Perforierter Vliesstoff nach einem der Ansprüche 1 bis 6, **dadurch gekennzeichnet, daß** der Strike Through-Wert nach einer Minute weniger als 3 Sekunden, der Rewet-Wert weniger als 0,5 9 und die Höchstzugkraft in Längsrichtung mindestens 30N/5 cm betragen.

8. Verfahren zur Herstellung von perforierten Vliesstoffen nach einem der Ansprüche 1 bis 7 durch Ablegen von splittbaren Pie- oder Hollow-Pie-Endlosfasern, deren Querschnitt mindestens zwei unterschiedliche thermoplastischePolymere mit unterschiedlicher Hydrophobizität in einer abwechselnden Kuchenstückanordnung aufweist, zu einem Vliesstoff, nachfolgendes Splitten und Verschlingen der **Splitt-Filamente** zu verschlungenen **Mikrofaserfilamenten** mit Hilfe von Hochdruck-Wasserstrahlen, und nachfolgendes Perforieren des gebildeten Vliesstoffes mit Hochdruck Wasserstrahlen.

9. Verfahren nach Anspruch 8, **dadurch gekennzeichnet, daß** das Perforieren auf Entwässungs- und Lochbildungstrommeln erfolgt, die Erhebungen auf der Oberfläche aufweisen.

10. Verwendung von perforiertem Vliesstoff nach einem der Ansprüche 1 bis 7 als Topsheet in Hygieneprodukten wie Windeln oder Damenbinden.

## Claims

1. An apertured nonwoven having a basis weight in the range from 8 to 17 g/m² comprising entangled continuous microfibre filaments having a linear density in the range from 0.05 to 0.40 dtex which are constructed of at least two thermoplastic polymers having different hydrophobicities and have a pie or hollow pie cross section, from which the split filaments have been released, the apertures being clearly defined and free from split fibre filaments.

2. An apertured nonwoven according to claim 1, **characterized in that** the apertures form a regular array and have an individual hole area in the range from 0.01 to 0.60 cm².

3. An apertured nonwoven according to claim 1 or 2, **characterized in that** in the nonwoven the ratio of the maximum distance of points on the nonwoven surface to the next aperture to the minimum distance is in the range from 1:1 to 2:1.

4. An apertured nonwoven according to any one of claims 1 to 3, **characterized in that** the open area is in the range from 8% to 40%.

5. An apertured nonwoven according to any one of claims 1 to 4, **characterized in that** the apertured nonwoven is constructed of polyolefin and polyester filaments in a weight ratio in the range from 20:80 to 80:20.

6. An apertured nonwoven according to any one of claims 1 to 5, **characterized in that** the nonwoven is impregnated with from 0% to 0.60% by weight, based on the nonwoven weight, of at least one surfactant.

7. An apertured nonwoven according to any one of claims 1 to 6, **characterized in that** the strikethrough value after one minute is less than 3 seconds, the rewet value is less than 0.5 g and the ultimate tensile strength in the machine direction is at least 30 N/5 cm.

8. A process for producing an apertured nonwoven according to any one of claims 1 to 7 by laying down splittable pie or hollow pie continuous fibres whose cross section comprises at least two different thermoplastic polymers having different hydrophobicities in an alternating segmented pie arrangement to form a nonwoven, subsequently splitting and entangling the split filaments to form entangled microfibre filaments by means of high pressure water jets and subsequent aperturing of the resultant nonwoven using high pressure water jets.

9. A process according to claim 8, **characterized in that** the aperturing is effected on drainage and hole-forming drums having protrusions on the surface.

10. Use of an apertured nonwoven according to any one of claims 1 to 7 as topsheet in hygiene products such as diapers or sanitary napkins.

## Revendications

1. Nappe de fibres perforée ayant un grammage compris entre 8 et 17 g/m² et composée de filaments de microfibres sans fin enchevêtrées entre elles et ayant un titre dans la gamme de 0,05 à 0,40 dtex, qui sont formés d'au moins deux polymères thermoplastiques présentant des hydrophobicités différentes, et qui présentent une section de filament en forme de camembert (dite "pie") ou de camembert creux (dite "hollow-pie") et dont les filaments éclatés (dits "splitt") ont été libérés, les perforations étant exécutées de façon nette et étant exemptes de filaments de fibres éclatés.

2. Nappe de fibres perforée selon la revendication 1, **caractérisée en ce que** les perforations sont disposées régulièrement et présentent une surface de trou individuelle comprise entre 0,01 et 0,60 cm².

3. Nappe de fibres perforée selon la revendication 1 ou 2, **caractérisée en ce que** dans la nappe de fibres, le rapport entre éloignement maximal et éloignement minimal des points de la surface de la nappe à la perforation suivante est compris entre 1:1 1 et 2:1.

4. Nappe de fibres perforée selon l'une des revendications 1 à 3, **caractérisée en ce que** la surface des trous ouverts forme 8 à 40 %.

5. Nappe de fibres perforée selon l'une des revendications 1 à 4, **caractérisée en ce que** la nappe de fibres perforée est fabriquée à partir de filaments réalisés en polyoléfine et en polyester selon un rapport de masse dans la gamme comprise entre 20:80 et 80:20.

6. Nappe de fibres perforée selon l'une des revendications 1 à 5, **caractérisée en ce que** la nappe de fibres est imprégnée de 0 à 0,60 % en masse, sur la base de la masse de la nappe de fibres, d'au moins une substance active tensioactive.

7. Nappe de fibres perforée selon l'une des revendications 1 à 6, **caractérisée en ce que** la valeur de pénétration (strike through) après une minute est inférieure à 3 secondes, la valeur de réhumidification (rewet) est inférieure à 0,59 et l'effort de traction maximal dans la direction longitudinale est d'au moins 30 N/5 cm.

8. Procédé de fabrication de nappes de fibres perforées selon l'une des revendications 1 à 7 par dépôt de fibres sans fin en forme de camembert (forme dite "pie") ou de camembert creux (forme dite "hollow-pie"), dont la section présente au moins deux polymères thermoplastiques différents présentant des hydrophobicités différentes, selon une disposition en parts de gâteaux alternées, pour former une nappe de fibres; éclatement ultérieur (splittage) et enchevêtrement des filaments éclatés (dits "splitt") en filaments de microfibres enchevêtrés à l'aide d'un jet d'eau sous haute pression ; et perforation ultérieure de la nappe de fibres formée avec des jets d'eau sous haute pression.

9. Procédé selon la revendication 8, **caractérisé en ce que** la perforation se produit sur des tambours de déshydratation et de perforation qui présentent des élévations sur leur surface.

10. Utilisation d'une nappe de fibres perforée selon l'une des revendications 1 à 7 en tant que feuille supérieure ou de couverture de produits d'hygiène comme des couches ou des serviettes hygiéniques.
